# NEW EUROPEAN PATENT SPECIFICATION

(11) **EP 0 706 788 B2**
(45) Date of publication and mention of the opposition decision: **28.01.2004**
(45) Mention of the grant of the patent: 29.03.2000
(21) Application number: 95111963.5
(22) Date of filing: 28.07.1995
(51) Int. Cl.: A61K 7/13

(54) **Acid dye containing colorants**
Säurefarbstoff enthaltende Zusammensetzungen
Compositions contenant des colorants acidiques

(30) Priority: 30.07.1994 JP 19786094; 30.07.1994 JP 19786194
(43) Date of publication of application: 17.04.1996
(73) Proprietor: KAO CORPORATION, Chuo-ku, Tokyo (JP)
(72) Inventor: Shibata, Hiroshi, c/o Kao Corporation Res. Lab., Tokyo (JP); Koga, Hiroyuki, c/o Kao Corporation Res. Lab., Tokyo (JP); Nagashima, Nozomi, c/o Kao Corporation Res. Lab., Tokyo (JP); Masumoto, Kazunori, c/o Kao Corporation Res. Lab., Tokyo (JP)
(74) Representative: Hansen, Bernd, Dr. Dipl.-Chem.

(56) References cited:
- EP-A- 0 312 343
- EP-A- 0 384 415
- EP-A- 0 470 381
- EP-A- 0 512 270
- EP-A- 0 531 943
- DE-A- 3 106 600
- DE-A- 3 837 473
- GB-A- 2 091 102
- US-A- 3 653 797
- Colloid & Polymer Science 269 (1992), pp. 85-90

## Description

### FIELD OF THE INVENTION

This invention relates to dyeing cosmetics. More particularly, it relates to colorants using an acid dye which are capable, by their repeated use, of making silver hairs inconspicuous, or coloring black hair to give a tonal difference or to take pleasure in delicately changed hair color, and which are excellent in their dyeing power and conditioning effect while hardly cause transfer of color to the head skin or hands and do not cause damage to hair or an eruption on the head skin, thus being safe. It also relates to a method for producing such colorants and the use thereof.

### BACKGROUND OF THE INVENTION

In recent years, consumers have been increasingly desirous of making silver hairs inconspicuous gradually as if they became black naturally, of giving black hair brightness little by little or of changing a nuance of hair color.

In order to comply with such wishes, hairdye articles have been commercialized which contain a small amount of basic dyes or acid dyes and can be used like a rinse.

However, the hairdye articles containing basic dyes merely exert their effect by temporary adsorption of the dyes to hair, and therefore the effect disappears after the subsequent shampooing. In addition, basic dyes are not suited for those articles which are treated by a bare hand because of their high adsorption power to the skin.

On the other hand, in the hairdye articles containing acid dyes, an anionic surfactant is used in order to give dispersibility of the dyes, and an aromatic alcohol-based permeation enhancing agent such as dibenzyl alcohol is used in combination with an organic acid for the purpose of effecting permeation of the acid dyes into hair at a low pH. The reason for the use of an anionic surfactant as a surfactant is that the dyeing power is considerably inhibited when a cationic surfactant is used, because acid dyes usually have negative charge and form complex bodies with the cationic surfactant so that the dyes cannot penetrate into hair.

However, the use of an anionic surfactant poses a problem in that conditioning effects insufficient so that smoothness and slipperiness of hair at the time of rinsing and drying becomes unsatisfactory.

A countermeasure has been proposed in which acid dyes are used in combination with a specific cationic surfactant to provide not only the dyeing power but also the conditioning effects such as pleasantness to the touch, smooth combing and the like (JP-A-5-43438; the term "JP-A" as used herein means an "unexamined published Japanese patent application"). The dyeing power, however, was not enough and the stability as a hairdye composition was not satisfactory.

On the other hand, JP-A-5-229919 proposes a hair cosmetic comprising an alkylamine oxide surfactant and an oily material having a low HLB in order to supplement conditioning effects of conventional acid dye-containing colorant; and JP-A-5-246831 proposes a colorant which contains a specific nonionic surfactant.

However, consumers still make a demand for a more higher dyeing power. The dyeing power may be improved by increasing concentration of dyes in colorants, but it causes a problem of stability reduction of the colorants.

Such a problem of reduced stability of colorants may be resolved by blending a relatively large amount of a cationic surfactant to effect re-dispersion of complexes, but increase in the amount of a cationic surfactant will entail another problem of considerably reduction in the dyeing power. On the other hand, when blending ratio of a nonionic surfactant such as polyoxyethylene alkyl ether or the like is increased in order to improve stability of colorants, dyes are incorporated into enlarged micelle of polyoxyethylene alkyl ether, hence also entailing a problem of causing reduction in the dyeing power.
EP-A-0 531 943 relates to a hair tinting shampoo which contains a surface-active amine oxide, an alkyl polyglycoside and a direct dyestuff such as Acid Yellow 1 or Acid Yellow 9.
EP-A-0 512 270 discloses a cosmetic composition which comprises
(A) a nonionic amphipathic compound having in the molecule thereof at least one long chain branched alkyl or alkenyl group and at least three hydroxyl groups, and having a lamellar liquid crystal structure itself at 25° C and at 50°C or higher; and
(B) a compound represented by formula (1): 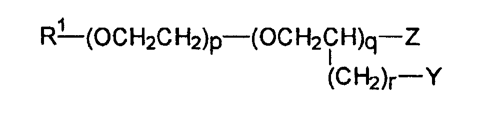
wherein R¹ is a hydrogen atom, a lower alkyl group or a group 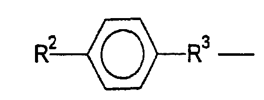 R² is a hydrogen atom, a methyl group or a methoxy group; R³ is a direct bond or a saturated or unsaturated divalent hydrocarbon group having 1 to 3 carbon atoms; Each Y and each Z are independently hydrogen atoms or hydroxyl groups; and p, q and r are each independently integers of 0 to 5, excepting the case where all of p, q and r are zero and Z is H, and the case where all of p, q and r are zero, R¹ is H and Z is OH.

### SUMMARY OF THE INVENTION

An object of the present invention is to provide a colorant, the dyeing power of which is increased by easy permeation of an acid dye into hair and is able to provide a markedly improved post-use feel.

The present inventors have found that the stability of the colorant is improved markedly when a specific amphiphilic compound is used in combination with a cationic surfactant or an amine oxide surfactant and an acid dye. The present inventors have found also that such a specific combination renders possible to attain not only easy permeation of the dye which formed a complex with the cationic surfactant or amine oxide surfactant into hair, hence increasing its dyeing power, but also considerable increase in the conditioning effects to improve feeling of hair after drying. The present invention has been accomplished on the basis of these findings.

Thus, according to the present invention, there is provided a colorant which comprises the following components (A), (B) and (C):
(A) a cationic surfactant and/or an amine oxide surfactant,
(B) a nonionic amphiphilic compound which forms a liquid crystal structure in an aqueous system at 0 to 50°C: selected from at least one of the compounds (B-1) to (B-5) as specified in claim 1
(C) an acid dye, and
(D) an aromatic alcobol

The present invention also provides a method for the production of such a colorant by mixing the components (A), (B), (C) and (D) and the use of such a colorant for dyeing and/or improving the texture of hair.

### DETAILED DESCRIPTION OF THE INVENTION

According to the present invention, a cationic surfactant or an amine oxide surfactant is used as Component
(A). By the use of the cationic or amine oxide surfactant, the sense of touch during and after use of the colorant is considerably improved in comparison with the case of the single use of an anionic or nonionic surfactant.

A compound represented by the following formula (I) may preferably be used as the cationic surfactant of Component (A).

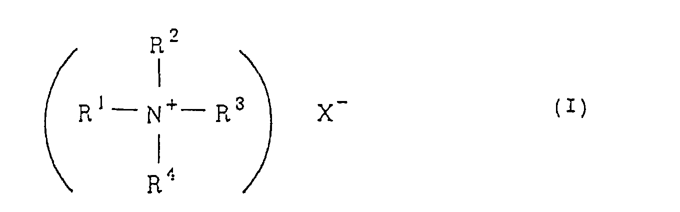

In the above formula, R¹ represents an alkyl or hydroxyalkyl group having 8 to 28 carbon atoms, R² represents an alkyl or hydroxyalkyl group having 1 to 4 carbon atoms, a benzyl group, a pyridyl group or an alkyl or hydroxyalkyl group having 8 to 22 carbon atoms, R³ and R⁴ may be the same or different from each other and each represents an alkyl or hydroxyalkyl group having 1 to 4 carbon atoms or a group represented by: -(CH₂-CH(R⁵)O)ₘ₁H (R⁵ is a hydrogen atom or an alkyl group having 1 to 4 carbon atoms and m1 = 1-20) and X represents a halogen atom or an alkyl sulfate group having 1 to 2 carbon atoms.

Specific examples of the cationic surfactant include lauryl trimethyl ammonium chloride, lauryl trimethyl ammonium bromide, myristyl trimethyl ammonium chloride, myristyl trimethyl ammonium bromide, cetyl trimethyl ammonium chloride, cetyl trimethyl ammonium bromide, stearyl trimethyl ammonium chloride, stearyl trimethyl ammonium bromide, behenyl trimethyl ammonium chloride, behenyl trimethyl ammonium bromide, cetyl trimethyl ammonium methane sulfonate, stearyl trimethyl ammonium methane sulfonate, myristyl dimethylbenzyl ammonium chloride, cetyl dimethylbenzyl ammonium chloride, stearyl dimethylbenzyl ammonium chloride, octyl dihydroxyethylmethyl ammonium chloride, distearyl dimethyl ammonium chloride, dicetyl dimethyl ammonium chloride, dicocoyl dimethy ammonium chloride, dialkyl(12-15) dimethyl ammonium chloride, dialkyl(14-18) dimethyl ammonium chloride, 2-decyltetradecyl trimethyl ammonium chloride, 2-dodecylhexadecyl dodecylhexadecyl trimethyl ammonium chloride, dimethyl ammonium chloride, di-(2-octyldodecyl) dimethyl ammonium chloride, dipolyoxyethylene(2) coconut oil alkyl methyl ammonium chloride, dipolyoxyethylene(15) coconut oil alkyl methyl ammonium chloride, hydroxyethylcetyl dimethyl ammonium chloride and the like.

A compound represented by the following formula (II) may preferably be used as the amine oxide surfactant of Component (A).

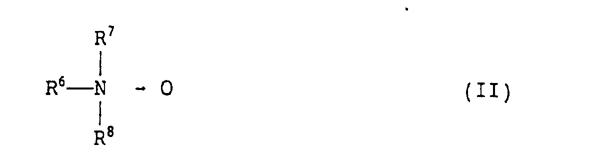

In the above formula, R⁶ represents an alkyl or hydroxyalkyl group having 8 to 22 carbon atoms or a group represented by: R⁹-(OCH(R¹⁰)-CH₂)ₘ₂- (R⁹ represents an alkyl or hydroxyalkyl group having 8 to 22 carbon atoms, R¹⁰ represents an alkyl group having 1 to 4 carbon atoms or a hydrogen atom and m2 = 0 to 3) and R⁷ and R⁸ may be the same or different from each other and each represents an alkyl or hydroxyalkyl group having 1 to 4 carbon atoms or a group represented by: -(CH₂-CH(R¹¹)O)ₙ₁H (R¹¹ is a hydrogen atom or an alkyl group having 1 to 4 carbon atoms and nl = 1 to 3).

Specific examples of the amine oxide surfactant include lauryl dimethylamine oxide, myristyl dimethylamine oxide, cetyl dimethylamine oxide, stearyl dimethylamine oxide, behenyl dimethylamine oxide, polyoxyethylene(3) coconut oil alkyl dimethylamine oxide, polyoxyethylene(3) laurylamine oxide, lauryl polyoxyethylene dimethylamine oxide and the like.

These cationic and amine oxide surfactants may be used alone or as a mixture of two or more in the colorant of the present invention.

The total amount of the cationic and amine oxide surfactants in the colorant may be within the range of generally from 0.01 to 10 % by weight, preferably from 0.1 to 5 % by weight, based on the weight of the colorant. If the amount is smaller than 0.01 % by weight, no sufficient effect on improvement in the sense of touch may be obtained, while if the amount is larger than 10 % by weight, no proportionally improvement in the effects may be obtained.

The nonionic amphiphilic compound to be used as Compound (B) in the present invention is a nonionic amphiphilic compound which forms a liquid crystal structure in an aqueous system at a temperature of from 0 to 50°C, preferably from 5 to 40°C, more preferably from 5 to 35°C, namely within a temperature range that includes general treating temperatures of colorants, or saccharide nonionic surfactants.

The nonionic amphiphilic compound which forms a liquid crystal structure has at least one branched long chain alkyl or alkenyl group per molecule. The use of a liquid crystal structure-forming amphiphilic compound having a branched long chain alkyl or alkenyl group is effective in improving the sense of touch when the colorant is used. The liquid crystal structure-forming amphiphilic compound contains at least two hydroxyl groups, because the liquid crystal structure can be easily formed thereby

The liquid crystal structure formed by the amphiphilic compound is a lamellar liquid crystal structure or a reversed middle liquid crystal structure. A compound which forms a lamellar liquid crystal structure is used because its molecules form a layer on hair, with their hydrophilic groups facing an acid dye and their lipophilic groups facing the hair side, so that the acid dye is effectively adhered to and permeated into hair. A compound which forms a reversed middle liquid crystal structure is also used because its molecules are arranged in a cylindrical form with their hydrophilic groups facing inside of the cylinder wall, so that an acid dye is incorporated into the cylindrical form and the lipophilic groups facing outside of the cylindrical form adhered to hair, thereby enhancing permeation of the acid dye into hair.

Formation of the lamellar liquid crystal structure can be confirmed by X-ray diffraction or using a differential scanning calorimeter (DSC) in accordance, for example, with the method disclosed in The Journal of Cell Biology, vol. 12, pp.207-209 or in Hyomen (Surface), vol.11, pp.579-590.

The liquid crystal structure-forming amphiphilic compounds which form a lamellar liquid crystal structure are selected from at least one of the compounds (B-1) to (B-4) which will be described below in detail.

### (B-1) Glycerylated polyols represented by the following formula (1):

Aₐ₁(G¹)

wherein G¹ represents a residue resulting from the elimination of a1 hydroxyl group(s) from a polyol selected from pentaerythritol, sorbitol, maltitol, glucose, fructose and an alkyl glycoside, A is a group represented by formula: 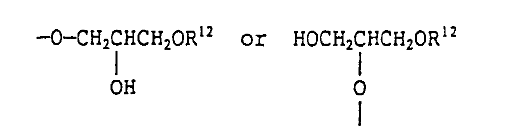 wherein R¹² represents a branched alkyl or alkenyl group having 10 to 36 carbon atoms, and a1 is a number of 1 or more which does not exceed the total number of hydroxyl groups of the aforementioned polyol.

In the glycerylated polyols (B-1), examples of the alkyl glycoside represented by G¹ in the above formula (1) include methyl glycoside, ethyl glycoside, propyl glycoside, octyl glycoside, methyl maltoside, ethyl maltoside and the like.

As R¹² in the group represented by A in the above formula (1), a branched alkyl group having 16 to 36 carbon atoms, particularly 18 to 24 carbon atoms, is preferred. Preferred examples of the branched alkyl group R¹² include those represented by the following formula (2) or (3): 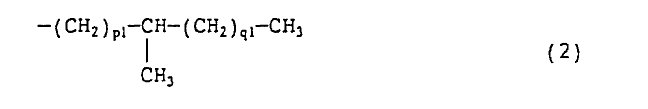 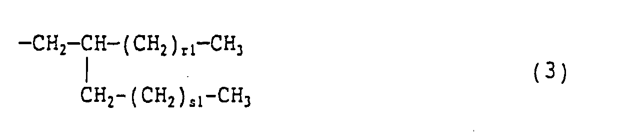 wherein each of p1 and q1 is an integer of 0 to 33 and the sum of p1 and q1 is 6 to 33, and each of r1 and s1 is an integer of 0 to 31 and the sum of r1 and s1 is 4 to 31.

Specific and preferred examples of the branched alkyl groups include methylpentadecyl, methylhexadecyl, methylheptadecyl (isostearyl), methyloctadecyl, methylbehenyl, ethylhexadecyl, ethyloctadecyl, ethylbehenyl, butyldodecyl, butylhexadecyl, butyloctadecyl, hexyldecyl, heptylundecyl, octyldodecyl, decyldodecyl, decyltetradecyl, dodecylhexadecyl, tetradecyloctadecyl and the like groups.

In the formula (1), a1 is particularly preferably 1 or 2.

### (B-2) Methyl branched fatty acid esters represented by the following formula (4):

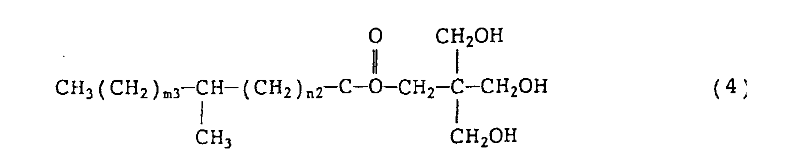

wherein each of m3 and n2 is an integer of 0 to 33 and the sum of m3 and n2 is 6 to 33.

In the above formula (4), the sum of m3 and n2 is 6 to 33, and preferably 10 to 16 in view of the easy adsorption of the dye to hair and the sense of touch when used.

### (B-3) Branched fatty acid glyceroglycolipids represented by the following formula (5):

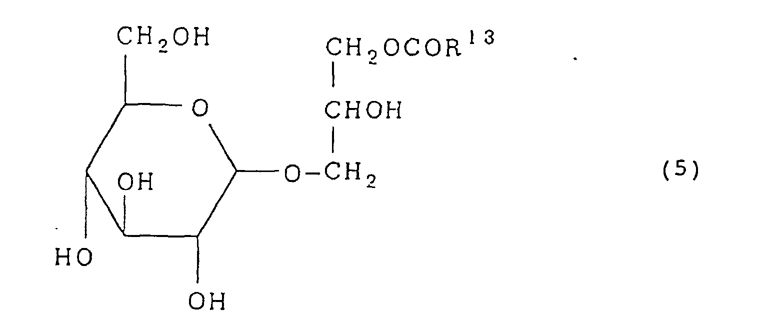

wherein R¹³ is a group represented by formula: 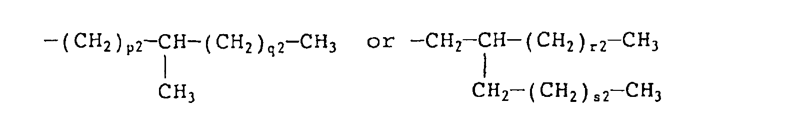 wherein each of p2 and q2 is an integer of 0 to 33 and the sum of p2 and q2 is 6 to 33, and each of r2 and s2 is an integer of 0 to 31 and the sum of r2 and s2 is 4 to 31.

In the above formula (5), similar to the case of m3 and n2 of the aforementioned formula (4), the total of p2 and q2 is preferably 10 to 16, more preferably 14, in view of the easy adsorption of the dye to hair and the sense of touch when used. From the same viewpoint, the sum of r2 and s2 is preferably 6 to 14, more preferably 8 to 12.

### (B-4) Alkyl trismethylols represented by the following formula (6) or alkyl trismethylol amides represented by the following formula (7):

R¹⁴-C(CH₂OH)₃ (6)

R¹⁴-CONHC(CH₂OH)₃ (7)

wherein R¹⁴ represents a branched alkyl group having 6 to 22 carbon atoms.

Each of the thus described compounds (B-1) to (B-4) is a thermotropic liquid crystal which maintains a lamellar liquid crystal structure by itself at a temperature of from 0 to 50°C in an aqueous system and has an excellent property
in that, when mixed with water, it disperses in water almost uniformly in the form of lamellar liquid crystals. In that case, the mixing ratio (weight ratio) of water and each of the compounds (B-1) to (B-4) may be within the range of from 99/1 to 1/99.

On the other hand, an α-mono(branched alkyl) glyceryl ether (B-5) represented by the following formula (8) may be used as the nonionic amphiphilic compound which forms a reversed middle liquid crystal structure.

R¹⁵-OCH₂CH(OH)CH₂OH (8)

In the above formula, R¹⁵ represents a branched saturated hydrocarbon group having 9 to 36 carbon atoms, preferably a methyl branched alkyl group represented by the following formula: 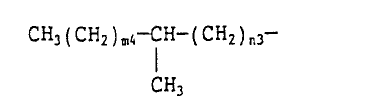 wherein each of m4 and n3 is an integer of 0 to 33 and the sum of m4 and n3 is 6 to 33.

The α-mono(methyl branched alkyl) glyceryl ether of formula (B) can be produced in accordance, for example, with the method disclosed in JP-B-61-26997 (corresponds to U.S. Patent 4,465,869, hereby incorporated by reference; the term "JP-B" as used herein means an "examined Japanese patent publication") or in JP-B-62-1368 (corresponds to U.S. Patent 4,486,406 and U.S. Patent 4,487,760, hereby incorporated by reference).

Similar to the case of the aforementioned compounds (B-1) to (B-4), the α-mono(methyl branched alkyl) glyceryl ether of formula (B) also forms a uniform reversed middle liquid crystal structure when mixed with water at a temperature of from 0 to 50°C. In that case, the mixing ratio (weight ratio) of water and the compound of formula (8) may be within the range of from 99/1 to 1/99.

These liquid crystal structure-forming amphiphilic compounds may be used alone or as a mixture of two or more.

The saccharide nonionic surfactant to be used as Component (B) of the present invention (e.g those which belong to the aforementioned compounds (B-1) and (B-3)) may be used in combination with the following alkyl saccharide surfactant (B'-1), sugar amide surfactant (B'-2) and sucrose fatty acid ester surfactant (B'-3).

### (B'-1) Alkyl saccharide surfactants represented by the following formula (9):

R¹⁶-O-(R¹⁷O)ₐ₂-G² _{b} (9)

wherein R¹⁶ represents a straight-chain or branched alkyl, alkenyl or alkylphenyl group having 6 to 22 carbon atoms, R¹⁷ represents an alkylene group having 2 to 4 carbon atoms, G² represents a reducing sugar having 5 or 6 carbon atoms, a2 is a number of 0 to 10 and b is a number of 1 to 10.

In the above formula (9), R¹⁶ is a straight-chain or branched alkyl, alkenyl or alkylphenyl group having 6 to 22 carbon atoms, and a straight-chain or branched alkyl group having 10 to 20 carbon atoms (decyl, lauryl, myristyl or the like group) is particularly preferred from the viewpoint of easy adhesion of the dye to hair. R¹⁷ is ethylene, propylene, trimethylene, butylene or the like group. The average polymerization degree a2 of the alkylene oxide is a number of 0 to 10, and preferably 0 to 3, more preferably 0, from the viewpoint of the emulsification property. The reducing sugar moiety G² in the formula (9) comprises a residue of a reducing sugar having 5 or 6 carbon atoms as the basic unit, preferred examples of which include glucose, galactose and fructose. The average polymerization degree b of the reducing sugar ranges from 1 to 10, and it is preferred that those having the polymerization degree of 1 to 4 are included in an amount of 80 % by weight based on the alkyl saccharide surfactants. If the polymerization degree of reducing sugar is too large, it may cause reduction in the emulsification property and the dyeing power.

### (B'-2) Sugar amide surfactants represented by the following formula (10):

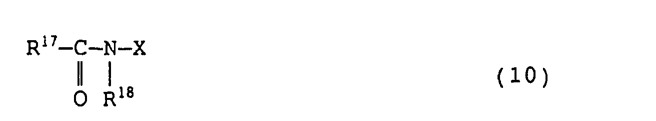

wherein R¹⁷ represents a straight-chain or branched alkyl, alkenyl or alkylphenyl group having 9 to 21 carbon atoms, R¹⁸ represents hydrogen atom, a straight-chain or branched alkyl or alkenyl group having 1 to 18 carbon atoms, a group represented by the following formula: 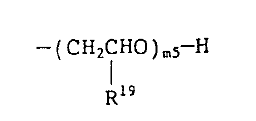 wherein R¹⁹ represents a hydrogen atom or a methyl group and m5 is a number of 0 to 10, - CH₂CH₂OH, -CH₂CH(OH)CH₃ or -CH₂CH₂CH₂OH, and X represents a polyhydroxyalkyl group which comprises a sugar residue having 4 to 30 carbon atoms.

In the above formula (10), R¹⁷ is a straight-chain or branched alkyl, alkenyl or alkylphenyl group having 9 to 21 carbon atoms, and its preferred examples are those in which R¹⁷-CO- is derived from capric acid, lauric acid, myristic acid, palmitic acid, stearic acid, isostearic acid, oleic acid or behenic acid, particularly those derived from palmitic acid or stearic acid. Specific examples of R¹⁸ include hydrogen atom, methyl group, ethyl group, n-propyl group, isopropyl group, n-butyl group, t-butyl group, n-hexyl group, octyl group, 2-ethylhexyl group, decyl group, dodecyl group, stearyl group, isostearyl group, a polyethylene glycol or polypropylene glycol group having a polymerization degree of 2 to 10, 2-hydroxyethyl group, 2-hydroxypropyl group, 3-hydroxypropyl group and the like, of which hydrogen atom, methyl group, ethyl group, 2-hydroxyethyl group, 2-hydroxypropyl group and 3-hydroxypropyl group are particularly preferred. X is a polyhydroxyalkyl group comprising a sugar residue having 4 to 30 carbon atoms, and examples thereof include polyhydroxyalkyl groups having 4 to 7 carbon atoms, each of which being linked to a mono-, di- or oligosaccharide group through glycoside linkage.

### (B'-3) Sucrose fatty acid ester surfactants represented by the following formula (11):

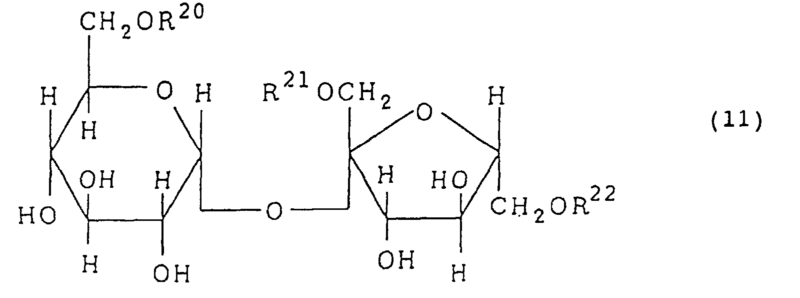

wherein R²⁰, R²¹ and R²² may be the same or different from one another and at least one of them represents a straight-chain or branched, saturated or unsaturated acyl group having 6 to 24 carbon atoms and the rest represents hydrogen atom(s).

In the above formula (11), at least one of R²⁰, R²¹ and R²² is an acyl group having 6 to 24 carbon atoms, and preferably 10 to 22 carbon atoms in view of higher emulsification property. The sucrose fatty acid ester surfactant generally comprises a mixture of mono-, di- and triester compounds, and it may have a monoester/di- or triester ratio of preferably from 70/30 to 30/70 in view of the stability of complexes in the colorant.

These saccharide nonionic surfactants may be used alone or as a mixture of two or more, when combined with a nonionic amphiphilic compound which forms a liquid crystal structure (component B).

The amount of the nonionic surfactant as Component (B) in the colorant of the present invention (including (B'-1) to (B'-3), if present) is preferably from 0.01 to 10 % by weight, more preferably from 0.1 to 10 % by weight, and furthermore preferably from 0.1 to 5 % by weight. If the amount is smaller than 0.01 %, no significant result may be obtained, while if the amount is larger than 10 % by weight, sticky sense of touch may be caused.

In the colorant of the present invention, an acid dye (component (C)) is used from the viewpoint of their gradual accumulation on hair and high safety. The acid dye can be selected optionally from known products depending on the desired color tone. Specific examples of the dye include Red No. 2 (C.I. No. 16185), Red No. 3 (C.I. No. 45430), Red No. 102 (C.I. No. 16255), Red No. 104 (C.I. No. 45410), Red No. 105 (C.I. No. 45440), Red No. 106 (C.I. No. 45100), Yellow No. 4 (C.I. No. 19140), Yellow No. 5 (C.I. No. 15985), Green No. 3 (C.I. No. 42053), Blue No. 1 (C.I. No. 42090), Blue No. 2 (C.I. No. 73015), Red No. 201 (C.I. No. 15850), Red No. 227 (C.I. No. 17200), Red No. 230 (C.I. No. 45380), Red No. 231 (C.I. No. 45410), Red No. 232 (C.I. No. 45440), Orange No. 205 (C.I. No. 15510), Orange No. 207 (C.I. No. 45425), Yellow No. 202 (C.I. No. 45350), Yellow No. 203 (C.I. No. 47005), Green No. 201 (C.I. No. 61570), Green No. 204 (C.I. No. 59040), Green No. 205 (C.I. No. 42095), Blue No. 202 (C.I. No. 42052), Blue No. 203 (C.I. No. 42052), Blue No. 205 (C.I. No. 42090), Brown No. 201 (C.I. No. 20170), Red No. 401 (C.I. No. 45190), Red No. 502 (C.I. No. 16155), Red No. 503 (C.I. No. 16150), Red No. 504 (C.I. No. 14700), Red No. 506 (C.I. No. 15620), Orange No. 402 (C.I. No. 14600), Yellow No. 402 (C.I. No. 18950), Yellow No. 403 (C.I. No. 10316), Yellow No. 406 (C.I. No. 13065), Yellow No. 407 (C.I. No. 18820), Green No. 401 (C.I. No. 10020), Green No. 402 (C.I. No. 42085), Purple No. 401 (C. I. No. 60730), Black No. 401 (C.I. No. 20470) and the like. Of these acid dyes, Yellow 4, Green 204, Red 2, Red 102, Green 3, Blue 1, Blue 205, Yellow 403, Red 106, Red 210, Orange 205, Black 401, Green 201 or Purple 401, particularly Black 401, Purple 401, Orange 205, Yellow 403 or Red 106, is preferred from the viewpoint of the hair dyeing power, safety and general applicability. These acid dyes may be used alone or as a mixture of two or more in the colorant of the present invention in an amount of preferably from 0.01 to 3 % by weight, more preferably from 0.08 to 2 % by weight, furthermore preferably from 0.08 to 1 % by weight, based on the weight of the colorant, depending on the desired color tone and dyeing power.

In addition to the aforementioned Components (A), (B), (C) and (D) the colorant of the present invention may further contain other alcohols, acids, organic solvents, pH adjusting agents, viscosity adjusting agents and the like.

The aromatic alcohol and the acid exert an effect of enhancing permeation of the acid dye into hair when they are used in combination.

The acid is preferably a weak acid because of the ion exchanging capacity of hair. Specific examples of the weak acid include citric acid, glycolic acid, succinic acid, tartaric acid, lactic acid, acetic acid, fumaric acid, malic acid, levulinic acid, butyric acid, valerianic acid, oxalic acid, maleic acid, mandelic acid, phosphoric acid and the like. It is preferred to use the weak acid in combination with its potassium salt, sodium salt and the like to constitute a buffer system. These acids may be used alone or as a mixture of two or more in an amount of preferably from 0.1 to 10 % by weight, more preferably from 0.5 to 7 % by weight, based on the weight of the colorant. In addition, these acids may be blended in such a manner that the colorant has a final pH value of preferably from 2 to 6, more preferably from 2.5 to 4.

Preferably, the aromatic alcohol may have a structure represented by the following formula (12): 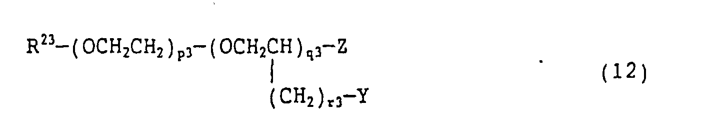 wherein R²³ is a group represented by the following formula: 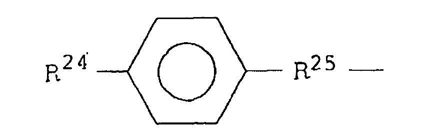 wherein R²⁴ represents hydrogen atom, methyl group or methoxy group and R²⁵ represents a bond or a saturated or unsaturated divalent hydrocarbon radical having 1 to 3 carbon atoms, each of Y and Z represents hydrogen atom or hydroxyl group and each of p3, q3 and r3 is a number of 0 to 5, provided that Z is not hydrogen atom when p3 = q3 = r3 = 0 and that Z is not hydroxyl group when p3 = q3 = r3 = 0 and R²³ = H.

Specific examples of the aromatic alcohol include benzyl alcohol, cinnamyl alcohol, phenethyl alcohol, p-anisyl alcohol, p-methylbenzyl alcohol, phenoxy ethanol, 2-benzyloxy ethanol and the like. These aromatic alcohols may be used alone or as a mixture of two or more in an amount of preferably from 1 to 20 % by weight, more preferably from 2 to 15 % by weight, based on the weight of the colorant.

The colorant of the present invention may further contain another known lower alcohols and lower polyols for improving the solubility of the aromatic alcohol in the colorant. Specific examples of the lower alcohol and the lower polyol include ethanol, isopropanol, n-propanol, n-butanol, ethylene glycol, propylene glycol, isoprene glycol, 1,3-butylene glycol, glycerol and the like. These alcohols may be used in an amount of from 1 to 40 % by weight based on the weight of the colorant.

The colorant of the present invention may further contain various cosmetically acceptable components used in conventional cosmetics so long as the effects of the present invention are not impaired. Examples of these components include anionic surfactants, amphoteric surfactants, nonionic surfactants such as polyoxyethylene alkyl ether and the like, cationic polymers such as cationic cellulose and the like, anionic polymers such as xanthan gum and the like, amphoteric polymers, water-soluble polymers such as hydroxyethyl cellulose and the like, oils such as paraffin oil, lanolin, silicone oil and the like, disinfectants, hair tonics, antiseptics, antidandruff agents, perfumes and the like. However, anionic surfactants may cause adverse effects on the uniformity and the stability of the composition, whereby impairing the effects of the present invention if it is present in a large amount, anionic surfactants should not be used in a large amount and preferably it is substantially excluded.

The colorant of the present invention may be produced by mixing the aforementioned various components in usual way and used in the same way as rinses.

Since the colorant of the present invention contain an acid dye, a cationic or amine oxide surfactant and a specific amphiphilic compound, stability of the complex formed from the acid dye, cationic or amine oxide surfactant and specific amphiphilic compound is improved, and the complex can easily permeate into hair. In consequence, the colorant of the present invention can show an excellent stability and dyeing power. In addition to this, high conditioning effects and excellent sense of touch during and after their use can be obtained.

The present invention will now be described in greater detail by referring to Examples, but the present invention is not restricted to these Examples.

### EXAMPLES 1 TO 7 AND COMPARATIVE EXAMPLES 1 TO 5

Colorants having respective compositions shown in Tables 1 and 2 were prepared and subjected to hair dyeing in accordance with the following dyeing method to carry out evaluation tests of their dyeing powers and conditioning effects. The results are shown in Tables 1 and 2.

### (Dyeing method)

A tress of goat hair having a weight of 1 g was washed with a commercially available shampoo liquid, rinsed thoroughly and then treated with a towel to wipe up excess water. Next, this was uniformly coated with about 0.3 g of each of the colorants of Examples and Comparative Examples, allowed to stand for 5 minutes, thoroughly rinsed with hot water and then dried. This treatment was repeated 3 times.

### (Dyeing power)

Color measurement was carried out using a color difference meter (CR200, manufactured by Minolta Camera Co., Ltd.) to calculate color difference (ΔE) from the original hair. A larger ΔE value means a higher dyeing power. The results were evaluated as A when the ΔE value was 20 or more, B when the value was less than 20 and 15 or more, and C when it was less than 15.

### (Conditioning effect)

The sense of touch after finishing was evaluated by 10 female panelists based on a five-step evaluation of: good (5 points), slightly good (4 points), usual (3 points), slightly bad (2 points) and bad (1 point). The results were evaluated as A when the average point was 4 points or more, B when it was less than 4 and 3 or more, C when it was less than 3 and 2 or more, and D when it was less than 2.

**TABLE 1**

| Amount (% by weight) | Examples | | | | | | |
|---|---|---|---|---|---|---|---|
| | 1 | 2 | 3 | 4 | 5 | 6 | 7 |
| Component (A) | | | | | | | |
| Stearyl trimethyl ammonium chloride | 0.3 | 0.3 | 0.3 | - | - | 0.3 | 0.15 |
| Cetyl dimethyl amine oxide | - | - | - | 0.3 | 0.3 | - | 0.15 |

| Component (B) | | | | | | | |
|---|---|---|---|---|---|---|---|
| Isostearyl glyceryl ether | 2 | - | - | 2 | - | 1 | 2 |
| Phytan Triol®*¹ | - | - | 2 | - | 2 | 1 | - |
| Pentaerythritol isostearyl glyceryl ether | - | 2 | - | - | - | - | - |

| Components (C) and (D) | | | | | | | |
|---|---|---|---|---|---|---|---|
| Dye (Orange No. 205) | 0.1 | 0.1 | 0.1 | 0.1 | 0.1 | 0.1 | 0.1 |
| Benzyloxy ethanol | 5 | 5 | 5 | 5 | 5 | 5 | 5 |
| Isoprene glycol | 10 | 10 | 10 | 10 | 10 | 10 | 10 |
| Lactic acid | 3 | 3 | 3 | 3 | 3 | 3 | 3 |
| Sodium hydroxide | 0.62 | 0.62 | 0.62 | 0.62 | 0.62 | 0.62 | 0.62 |
| Hydroxyethyl cellulose | 1 | 1 | 1 | 1 | 1 | 1 | 1 |
| Purified water | balance | balance | balance | balance | balance | balance | balance |
| pH (10 % aq.) | 3.8 | 3.8 | 3.9 | 4.0 | 4.2 | 3.9 | 3.9 |
| Dyeing power | A | A | A | A | A | A | A |
| Conditioning effect | A | A | B | A | B | B | A |
| Stability | A | A | B | A | A | B | A |

| | | | | | | | |
|---|---|---|---|---|---|---|---|
| *1: tetrametyltrihydroxyhexadecane (manufactured by Kuraray Co., Ltd.) | | | | | | | |

**TABLE 2**

| Amount (% by weight) | Comparative Examples | | | | |
|---|---|---|---|---|---|
| | 1 | 2 | 3 | 4 | 5 |
| Component (A) | | | | | |
| Stearyl trimethyl ammonium chloride | 0.3 | - | - | - | - |
| Cetyl dimethyl amine oxide | - | - | - | - | - |

| Component (B) | | | | | |
|---|---|---|---|---|---|
| Isostearyl glyceryl ether | - | 2 | - | 2 | - |
| Phytan Triol®*¹ | - | - | - | - | 2 |
| Pentaerythritol isostearyl glyceryl ether | - | - | - | - | - |

| Components (C) and (D) | | | | | |
|---|---|---|---|---|---|
| Dye (Orange No. 205) | 0.1 | 0.1 | 0.1 | 0.1 | 0.1 |
| Sodium hexadecyl sulfate | - | - | - | 0.3 | - |
| Sodium dodecyl sulfate | - | - | - | - | 0.3 |
| Benzyloxy ethanol | 5 | 5 | 5 | 5 | 5 |
| Isoprene glycol | 10 | 10 | 10 | 10 | 10 |
| Lactic acid | 3 | 3 | 3 | 3 | 3 |
| Sodium hydroxide | 0.62 | 0.62 | 0.62 | 0.62 | 0.62 |
| Hydroxyethyl cellulose | 1 | 1 | 1 | 1 | 1 |
| Purified water | balance | balance | balance | balance | balance |
| pH (10 % aq.) | 3.8 | 3.9 | 4.0 | 3.9 | 3.9 |
| Dyeing power | C | A | A | A | A |
| Conditioning effect | C | D | D | C | D |
| Stability | D | A | A | B | C |

As is evident from the results shown in Tables 1 and 2, each of the colorants of Examples 1 to 7 which contains both of Components (A) and (B) in addition to the acid dye of Component (C) is excellent in its dyeing power and conditioning effect, whereas Comparative Example 2 which lacks Component (A) is inferior in its conditioning effect and Comparative Example 1 which lacks Component (B) is inferior in its dyeing power. Also, Comparative Example 3 which lacks both Components (A) and (B) is inferior in its conditioning effect, and Comparative Examples 4 and 5 in which an anionic surfactant is used instead of Component (A) are poor in their conditioning effect and stability.

### EXAMPLES 8 TO 11 AND COMPARATIVE EXAMPLES 6 - 10

Colorants having respective compositions shown in Tables 3 and 4 were prepared and subjected to evaluation tests of their stabilities, dyeing powers and conditioning effects. The results are shown in Tables 3 and 4.

### (Stability)

A 100 g portion of each of the colorants was put into a sample bottle and stored at 30°C to observe the presence of separation with the naked eye. The results were evaluated as follows.
A: stable for 1 month or more
B: stable for 1 week or more
C: stable for 3 days or more
D: separation within 2 days

### (Dyeing power)

A tress of silver hair having a weight of 1 g was washed with a commercially available shampoo liquid, rinsed thoroughly and then treated with a towel to wipe up excess water. Next, this was uniformly coated with about 0.3 g of each of the colorants of Examples and Comparative Example, allowed to stand for 5 minutes, thoroughly rinsed with warm water and then dried.

Color measurement of the sample was carried out using a color difference meter (CR200, manufactured by Minolta Camera Co., Ltd.) to calculate color difference (ΔE) from the original silver hair. A larger ΔE value means a higher dyeing power. The results were evaluated as follows.
A: 30 or more
B: 20 or more but less than 30
C: 10 or more but less than 20
D: less than 10

### (Conditioning effect)

Evaluation was carried out in the same manner as described in the foregoing.

**TABLE 3**

| Amount (% by weight) | Examples | | | |
|---|---|---|---|---|
| | 8 | 9 | 10 | 11 |
| Component (A) | | | | |
| Stearyl trimethyl ammonium chloride | 1 | - | 1 | 1 |
| Cetyl dimethyl amine oxide | - | 1 | - | - |

| Component (B) | | | | |
|---|---|---|---|---|
| Stearyl polyglucoside | 5 | 5 | - | - |
| Isostearyl glyceryl ether | 4 | 4 | 4 | 4 |
| Palmitic acid sucrose ester | - | - | 5 | - |
| Stearyl maltoside | - | - | - | 2.9 |
| Palmityl maltoside | - | - | - | 3.0 |

| Components (C) and (D) | | | | |
|---|---|---|---|---|
| Dye (Orange No. 205) | 0.3 | 0.3 | 0.3 | 0.3 |
| Benzyloxy ethanol | 5 | 5 | 5 | 5 |
| Isoprene glycol | 10 | 10 | 10 | 10 |
| Lactic acid | 3 | 3 | 3 | 3 |
| Sodium hydroxide | 0.62 | 0.62 | 0.62 | 0.62 |
| Hydroxyethyl cellulose | 1 | 1 | 1 | 1 |
| Purified water | balance | balance | balance | balance |
| pH (10 % aq.) | 4.0 | 4.0 | 4.0 | 4.0 |
| Dyeing power | A | A | A | A |
| Conditioning effect | A | A | A | A |
| Stability | A | A | A | A |

**TABLE 4**

| Amount (% by weight) | Comparative Examples | | | | |
|---|---|---|---|---|---|
| | 6 | 7 | 8 | 9 | 10 |
| Component (A) | | | | | |
| Stearyl trimethyl ammonium chloride | 0.9 | - | 0.45 | 0.90 | - |
| Cetyl dimethyl amine oxide | - | 0.9 | 0.45 | - | - |

| Component (B) | | | | | |
|---|---|---|---|---|---|
| Stearyl polyglucoside | 3 | 3 | 3 | 12 | 3 |
| Isostearyl glyceryl ether | - | - | - | - | - |

| Components (C) and (D) | | | | | |
|---|---|---|---|---|---|
| Dye (Orange No. 205) | 0.2 | 0.2 | 0.2 | 0.2 | 0.2 |
| Benzyloxy ethanol | 5 | 5 | 5 | 5 | 5 |
| Isoprene glycol | 11 | 11 | 11 | 11 | 11 |
| Lactic acid | 3 | 3 | 3 | 3 | 3 |
| Sodium hydroxide | 0.62 | 0.62 | 0.62 | 0.62 | 0.62 |
| Hydroxyethyl cellulose | 0.5 | 0.5 | 0.5 | 0.5 | 0.5 |
| Purified water | balance | balance | balance | balance | balance |
| pH (10 % aq.) | 3.9 | 4.0 | 4.0 | 3.9 | 4.0 |
| Dyeing power | B | B | B | B | A |
| Conditioning effect | A | A | A | B | C |

As is evident from the results shown in Table 3, each of the colorants of Examples 8 to 11 which contains both of Components (A) and (B) in addition to the acid dye of Component (C) is excellent in its stability, dyeing power and conditioning effect, but Comparative Example 10 which lacks Component (A) is inferior in its conditioning effect.

As has been described in the foregoing, according to the present invention, the colorant having a high stability and dyeing power with markedly improved post-use touch of touch can be obtained.

## Claims

1. A colorant which comprises the following components (A), (B) , (C) and (D) :
(A) a cationic surfactant and/or an amine oxide surfactant,
(B) a nonionic amphiphilic compound which forms a liquid crystal structure in an aqueous system at 0 to 50°C, wherein said amphiphilic compound is selected from at least one of the following compounds (B-1) to (B-5) :
(B-1) Glycerylated polyols represented by the following formula (1) :
Aₐ₁(G¹) (1)
wherein G¹ represents a residue resulting from the elimination of a1 hydroxyl group(s) from a polyol selected from pentaerythritol, sorbitol, maltitol, glucose, fructose and an alkyl glycoside, A is a group represented by formula: 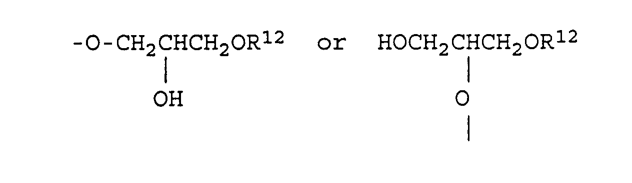 wherein R¹² represents a branched alkyl or alkenyl group having 10 to 36 carbon atoms, and a1 is a number of 1 or more which does not exceed the total number of hydroxyl groups of the aforementioned polyol,
(B-2) Methyl branched fatty acid esters represented by the following formula (4): 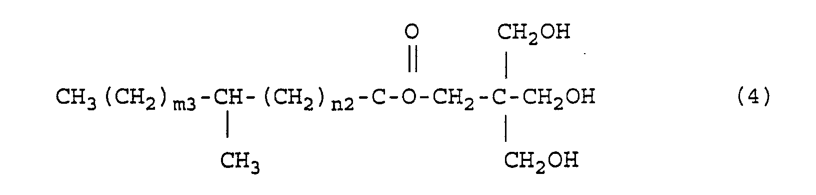 wherein each of m3 and n2 is an integer of 0 to 33 and the sum of m3 and n2 is 6 to 33,
(B-3) Branched fatty acid glyceroglycolipids represented by the following formula (5): 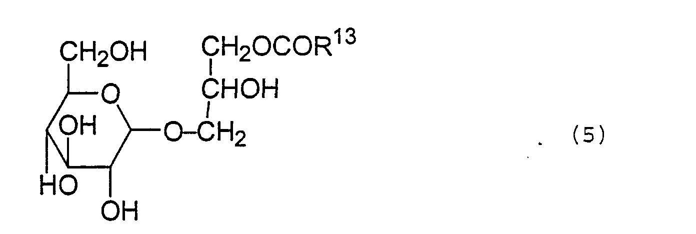 wherein R¹³ is a group represented by formula 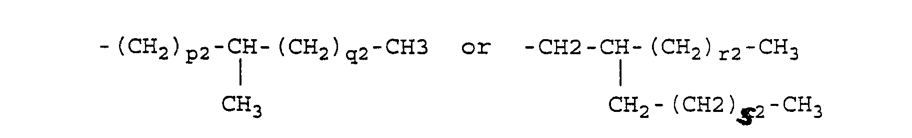 wherein each of p2 and q2 is an integer of 0 to 33 and the sum of p2 and q2 is 6 to 33, and each of r2 and s2 is an integer of 0 to 31 and the sum of r2 and s2 is 4 to 31,
(B-4) Alkyl trismethylols represented by the following formula (6) or alkyl trismethylol amides represented by the following formula (7)
R¹⁴-C(CH₂OH)₃ (6)
R¹⁴-CONHC(CH₂OH)₃ (7)
wherein R¹⁴ represents a branched alkyl group having 6 to 22 carbon atoms, or
(B-5) an α-mono (branched alkyl) glyceryl ether represented by the following formula (8)
R¹⁵-OCH₂CH(OH)CH₂OH (8)
wherein R¹⁵ represents a branched saturated hydrocarbon group having 9 to 36 carbon atoms,
(C) an acid dye, and
(D) an aromatic alcohol.

2. The colorant of claim 1, wherein the aromatic alcohol (D) has a structure represented by the following formula (12) : 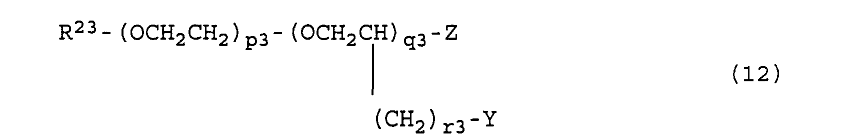 wherein R²³ is a group represented by the following formula : 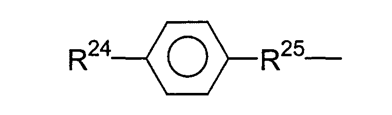 wherein R²⁴ represents hydrogen atom, methyl group or methoxy group and R²⁵ represents a bond or a saturated or unsaturated divalent hydrocarbon radical having 1 to 3 carbon atoms,
each of Y and Z represents hydrogen atom or hydroxyl group and each of p3, q3 and r3 is a number of 0 to 5, provided that Z is not hydrogen atom when p3=q3=r3=0 and that Z is not hydroxyl group when p3=q3=r3=0 and R²³=H.

3. The colorant of claim 1 or 2, wherein the aromatic alcohol (D) is present in an amount of 1 to 20% by weight, based on the weight of the colorant.

4. The colorant of claims 1-3, which further comprises a saccharide nonionic surfactant.

5. The colorant of claim 1, wherein Component (A) is present in an amount from 0.01 to 10% by weight, Component (B) is present in an amount of from 0.01 to 10% by weight and Component (C) is present in an amount of from 0.01 to 3% by weight.

6. The colorant of any of claims 1-5, which further comprises an acid.

7. The colorant of any of claims 1-6, which has a pH value of f rom 2 to 6.

8. A method for the production of a colorant of any one of claims 1-7 wherein the components (A), (B), (C) and (D) as defined in claim 1 are mixed.

9. A method for the production of a colorant according to claim 8, wherein an acid is additionally mixed.

10. A method for the production of a colorant according to claim 9, wherein said colorant has or is adjusted to a pH value of from 2 to 6.

11. The use of the colorant of any one of claims 1-7 as a cosmetic suitable for dying and/or improving the texture of hair.

## Patentansprüche

1. Färbemittel, umfassend die folgenden Komponenten (A), (B) und (C) :
(A) ein kationisches Tensid und/oder ein Aminoxid-Tensid,
(B) eine nichtionische, amphiphile Verbindung, die in einem wäßrigen System bei 0 bis 50°C eine Flüssigkristallstruktur bildet, worin die amphiphile Verbindung ausgewählt ist aus zumindest einer der folgenden Verbindungen (B-1) bis (B-5):
(B-1) glycerylierten Polyolen mit der folgenden Formel (1) :
Aₐ₁(G¹)
worin G¹ ein Rest ist, der von der Eliminierung von a1 Hydroxylgruppen von einem Polyol resultiert, ausgewählt aus Pentaerythrit, Sorbit, Maltit, Glucose, Fructose und einem Alkylglycosid, A eine Gruppe ist, dargestellt durch die Formel 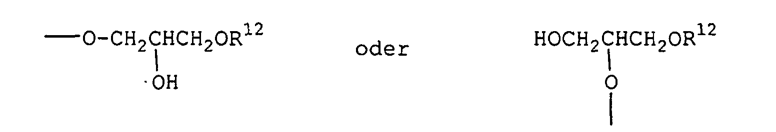 worin R¹² eine verzweigte Alkyl- oder Alkenylgruppe mit 10 bis 36 Kohlenstoffatomen und a1 eine Zahl von 1 oder mehr ist, die die Gesamtzahl der Hydroxylgruppen des erwähnten Polyols nicht übersteigt;
(B-2) Methyl-verzweigte Fettsäureester mit der folgenden Formel (4) 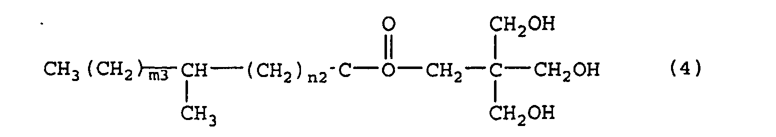 worin m3 und n2 jeweils eine ganze Zahl von 0 bis 33 und die Summe von m3 und n2 6 bis 33 ist;
(B-3) verzweigte Fettsäureglyceroglycolipide mit der folgenden Formel (5) 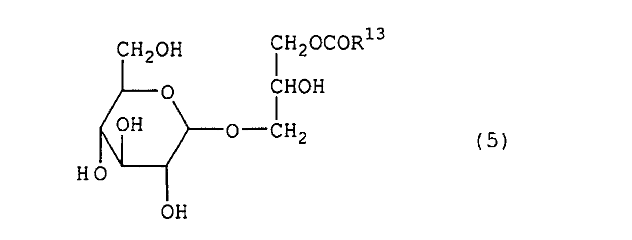 worin R¹³ eine Gruppe mit der Formel ist 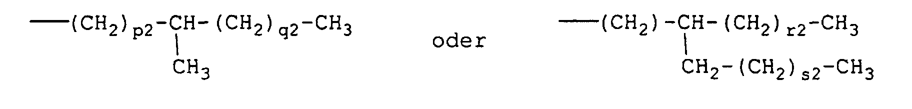 worin p2 und q2 jeweils eine ganze Zahl von 0 bis 33 und die Summe von p2 und q2 6 bis 33 ist und r2 und s2 jeweils eine ganze Zahl von 0 bis 31 und die Summe von r2 und s2 4 bis 31 sind;
(B-4) Alkyltrismethylole mit der folgenden Formel (6) oder Alkyltrismethylolamide mit der folgenden Formel (7)
R¹⁴-C(CH₂OH)₃ (6)
R¹⁴-CONHC(CH₂OH)₃ (7)
worin R¹⁴ eine verzweigte Alkylgruppe mit 6 bis 22 Kohlenstoffatomen ist, oder
(B-5) α-mono (verzweigtes Alkyl)glycerylether mit der folgenden Formel (8)
R¹⁵-OCH₂CH(OH)CH₂OH (8)
worin R¹⁵ eine verzweigte, gesättigte Kohlenwasserstoffgruppe mit 9 bis 36 Kohlenstoffatomen ist;
(C) einen sauren Farbstoff; und
(D) einen aromatischen Alkohol.

2. Färbemittel nach Anspruch 1, worin der aromatische Alkohol (D) eine Struktur mit der folgenden Formel (12) aufweist: 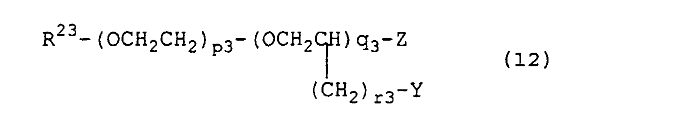 worin R²³ eine Gruppe mit der folgenden Formel ist: 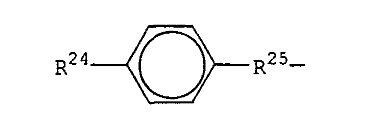 worin R²⁴ Wasserstoffatom, Methylgruppe oder Methoxygruppe ist und R²⁵ eine Bindung oder ein gesättigtes oder ungesättigtes bivalentes Kohlenwasserstoffradikal mit 1 bis 3 Kohlenstoffatomen ist, Y und Z jeweils ein Wasserstoffatom oder eine Hydroxylgruppe sind und p3, q3 und r3 jeweils eine Zahl von 0 bis 5 sind, vorausgesetzt, daß Z nicht Wasserstoff ist, wenn p3=q3=r3=0 und daß Z nicht Hydroxylgruppe ist, wenn p3=q3=r3=0 und R²³=H.

3. Färbemittel nach Anspruch 1 oder 2, worin der aromatische Alkohol (D) in einer Menge von 1 bis 20 Gew.-% vorhanden ist, bezogen auf das Gewicht des nicht ionischen Tensides.

4. Färbemittel nach den Ansprüchen 1 bis 3, das weiterhin ein nichtionisches Saccharid-Tensid enthält.

5. Färbemittel nach Anspruch 1, worin die Komponente (A) in einer Menge von 0,01 bis 10 Gew.-%, die Komponente (B) in einer Menge von 0,01 bis 10 Gew.-% und die Komponente (C) in einer Menge von 0,01 bis 3 Gew.-% vorhanden ist.

6. Färbemittel nach einem der Ansprüche 1 bis 5, die weiterhin eine Säure umfaßt.

7. Färbemittel nach einem der Ansprüche 1 bis 6, die einen pH-Wert von 2 bis 6 hat.

8. Verfahren zur Erzeugung eines Färbemittels nach einem der Ansprüche 1 bis 7, worin die Komponenten (A), (B), (C) und (D), die wie in Anspruch 1 definiert sind, gemischt werden.

9. Verfahren zur Erzeugung eines Färbemittels nach Anspruch 8, worin eine Säure zusätzlich vermischt wird.

10. Verfahren zur Herstellung eines Färbemittels nach Anspruch 9, worin das Färbemittel einen pH-Wert von 2 bis 6 hat und/oder der pH-Wert darauf eingestellt wird.

11. Verwendung des Färbemittels nach einem der Ansprüche 1 bis 7 als Kosmetikum, das zum Färben und/oder Verbessern der Haartextur geeignet ist.

## Revendications

1. Colorant comprenant les composants (A), (B) et (C) suivants:
(A) un agent tensioactif cationique et/ou un agent tensioactif d'oxyde aminé,
(B) un composé amphiphile non-ionique qui forme une structure de cristal liquide dans un système aqueux à 0 à 50 °C, dans lequel ledit composé amphiphile est choisi parmi l'un au moins des composés (B - 1) à (B - 5) suivants :
(B - 1) Des polyols glycérylés représentés par la formule (1) suivante :
Aₐ₁(G¹) (1)
dans laquelle G¹ représente un résidu résultant de l'élimination de a1 groupes hydroxyle d'un polyol choisi parmi le pentaérythritol, le sorbitol, le maltitol, le glucose, le fructose et un glycoside d'alkyle, A est un groupe représenté par la formule 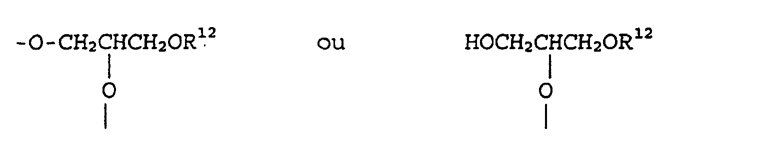 dans laquelle R¹² représente un groupe alkyle ou alcényle ramifié comptant de 10 à 36 atomes de carbone, et al représente un nombre de 1 ou plus, qui ne dépasse pas le nombre total des groupes hydroxyle du polyol susmentionné,
(B-2) Des esters méthyliques d'acides gras ramifiés représentés par la formule (4) suivante : 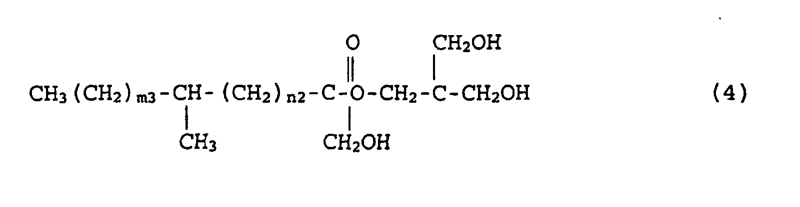 dans laquelle chacun parmi m3 et n2 est un entier de 0 à 33, et la somme de m3 et n2 vaut de 6 à 33,
(B-3) Des glycéroglycolipides d'acides gras ramifiés représentés par la formule (5) suivante : 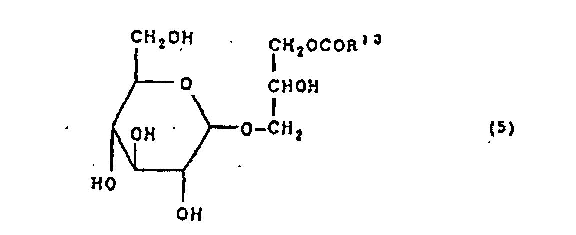 dans laquelle R¹³ est un groupe représenté par la formule 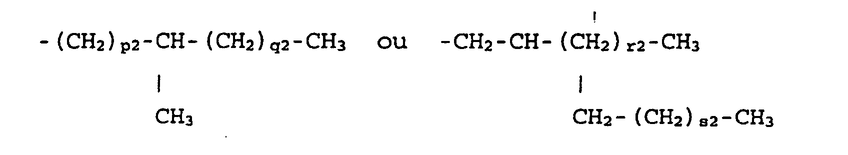 dans laquelle chacun parmi p2 et q2 est un entier de 0 à 33 et la somme de p2 et q2 vaut de 6 à 33, et chacun parmi r2 et S2 est un entier de 0 à 31, et la somme de r2 et s2 vaut 4 à 31,
(B-4) Des trisméthylols alkyliques représentés par la formule (6) suivante, ou des amides de trisméthylols alkyliques représentés par la formule (7) suivante
R¹⁴-C(CH₂OH)₃ (6)
R¹⁴-CONHC(CH₂OH)₃ (7)
dans lesquelles R¹⁴ représente un groupe alkyle ramifié comptant 6 à 22 atomes de carbone, ou
(B-5) un éther -mono (alkyle ramifié) glycérylique représenté par la formule (8) suivante
R¹⁵-OCH₂CH (OH) CH₂OH (8)
dans laquelle R15 représente un groupe d'hydrocarbure ramifié saturé comptant de 9 à 36 atomes de carbone,
(C) une matière colorante acide, et
(D) un alcool aromatique

2. Colorant selon la revendication 1, dans lequel l'alcool aromatique (D) a une structure représentée par la formule (12) suivante: 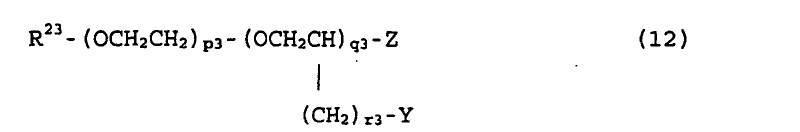 dans laquelle R²³ est un groupe représenté par la formule suivante : 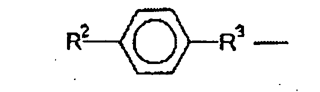 dans laquelle R²⁴ représente un atome d'hydrogène, un groupe méthyle ou un groupe méthoxy, et R²⁵ représente une liaison ou un groupe d'hydrocarbure bivalent, saturé ou insaturé, comptant 1 à 3 atomes de carbone,
chacun parmi Y et Z représente un atome d'hydrogène ou un groupe hydroxyle, et chacun parmi p3, q3 et r3 est un nombre de 0 à 5, avec la condition que Z n'est pas un atome d'hydrogène lorsque p3 = q3 = r3 = 0, et que Z n'est pas un groupe hydroxyle lorsque p3 = q3 = r3 = 0 et R²³ = H.

3. Colorant selon la revendication 1 ou 2, dans lequel l'alcool aromatique (D) est présent dans une quantité de 1 à 20% en poids, sur base du poids du colorant.

4. Colorant selon les revendications 1-3, qui comprend de plus un agent tensioactif non-ionique de saccharide.

5. Colorant selon la revendication 1, dans lequel le composant (A) est présent en une quantité de 0,01 à 10% en poids, le composant (B) est présent en une quantité de 0,01 à 10% en poids et le composant (C) est présent en une quantité de 0,01 à 3% en poids.

6. Colorant selon l'une quelconque des revendications 1 - 5, qui comprend de plus un acide.

7. Colorant selon l'une quelconque des revendications 1 - 6, qui présente une valeur de pH de 2 à 6.

8. Procédé de production d'un colorant selon l'une quelconque des revendications 1 à 7, dans lequel les composants (A), (B) et (C) et (D) tels que définis dans la revendication 1 sont mélangés.

9. Procédé de production d'un colorant selon la revendication 8, dans lequel un acide est additionnellement mélangé.

10. Procédé de production d'un colorant selon la revendication 9, dans lequel ledit colorant présente une valeur de pH de 2 à 6, ou est ajusté à une telle valeur.

11. Utilisation du colorant selon l'une quelconque des revendications de 1 à 7, en tant que cosmétique convenant pour colorer et/ou améliorer la texture du cheveu.
